(19) Europäisches Patentamt
European Patent Office
Office européen des brevets

(11) **EP 2 986 332 B1**

(12) **EUROPÄISCHE PATENTSCHRIFT**

(45) Veröffentlichungstag und Bekanntmachung des Hinweises auf die Patenterteilung:
**07.06.2017 Patentblatt 2017/23**

(21) Anmeldenummer: **14721756.6**

(22) Anmeldetag: **14.04.2014**

(51) Int Cl.:
**A61M 1/36** (2006.01)   **A61M 1/10** (2006.01)

(86) Internationale Anmeldenummer:
**PCT/EP2014/000997**

(87) Internationale Veröffentlichungsnummer:
**WO 2014/170007 (23.10.2014 Gazette 2014/43)**

(54) **VERFAHREN ZUR ERMITTLUNG DES DRUCKES IN EINEM EXTRAKORPORALEN KREISLAUF**

METHOD FOR DETERMINING THE PRESSURE IN AN EXTRACORPOREAL CIRCUIT

PROCÉDÉ DE DÉTERMINATION DE LA PRESSION DANS UNE CIRCULATION EXTRACORPORELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.04.2013 DE 102013006562**

(43) Veröffentlichungstag der Anmeldung:
**24.02.2016 Patentblatt 2016/08**

(73) Patentinhaber: **Fresenius Medical Care Deutschland GmbH**
**61352 Bad Homburg (DE)**

(72) Erfinder:
• **GAGEL, Alfred**
**96123 Litzendorf (DE)**
• **GLASER, Benedict**
**97422 Schweinfurt (DE)**
• **IBRAHIM, Haitham**
**97456 Dittelbrunn (DE)**
• **KOPPERSCHMIDT, Pascal**
**97456 Dittelbrunn (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
WO-A1-2009/144522     DE-A1-102009 027 195
DE-A1-102010 011 798

**Beschreibung**

Die vorliegende Erfindung betrifft ein Verfahren zur Ermittlung des Drucks oder eines mit dem Druck korrelierten Parameters in einem extrakorporalen Blutkreislauf einer Blutbehandlungsvorrichtung, insbesondere eines Dialysegerätes, wobei sich in dem Blutkreislauf wenigstens eine Blutpumpe befindet, die durch wenigstens einen Elektromotor angetrieben wird.

Die aus dem Stand der Technik bekannten Dialysevorrichtungen weisen üblicherweise einen arteriellen Zugang, einen venösen Zugang sowie eine Blutpumpe im extrakorporalen Blutkreislauf auf, mittels derer das Blut durch den extrakorporalen Kreislauf von dem arteriellen Zugang zu dem venösen Zugang und durch einen zwischen diesen angeordneten Dialysator gefördert wird.

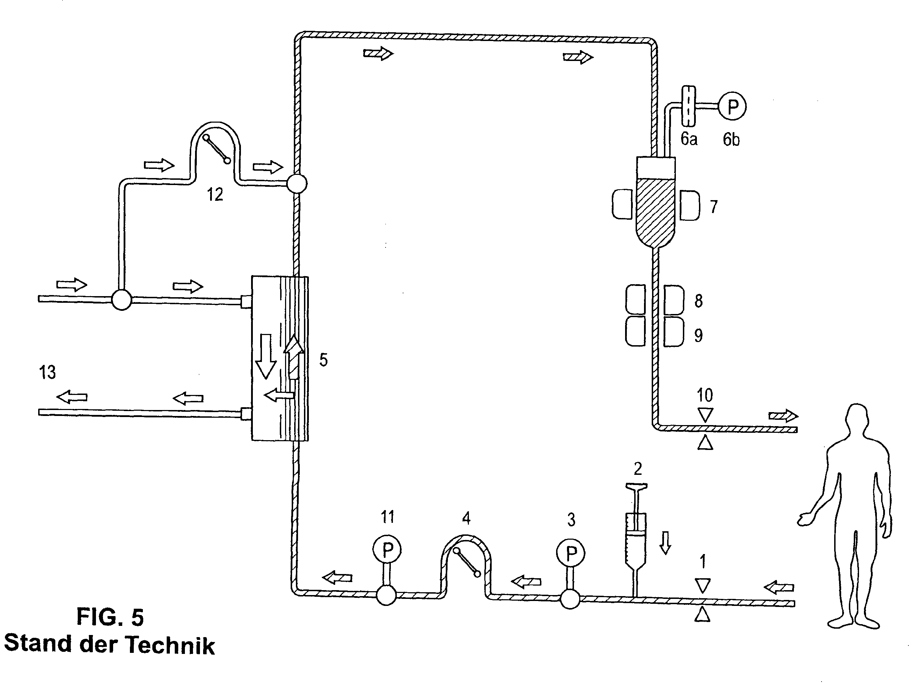

Ein derartiger, bekannter extrakorporaler Blutkreislauf ist beispielsweise in Figur 5 wiedergegeben.

Das Blut gelangt von dem Patienten aus dem arteriellen Zugang durch die arterielle Schlauchabsperrklemme 1 und über einen Anschluss für eine Heparin-Pumpe 2 zu der arteriellen Druckmesseinheit 3. In dieser Druckmesseinheit 3 wird der arterielle Druck $p_{art}$ ermittelt. Das Blut wird durch die Blutpumpe 4 gefördert, bei der es sich üblicherweise um eine peristaltische Pumpe handelt, die von einem Elektromotor angetrieben wird.

Das Blut gelangt nach Passieren der Pumpe 4 in einen Dialysator 5, wobei zwischen der Pumpe 4 und dem Dialysator 5 ein Pre-Filter oder Post-Pump-Drucksensor 11 zur Ermittlung des Druckwertes $p_{pp}$ angeordnet ist.

Stromabwärts des Dialysators 5 befindet sich eine Zufuhrleitung, über die mittels einer Substituatpumpe 12 eine Substitutionsflüssigkeit in das gereinigte Blut geleitet wird.

Der Dialysator 5 weist eine Blutkammer sowie eine Kammer für die Dialysierflüssigkeit auf, wobei diese im Gegenstrom durchströmt werden, wie dies durch die Pfeile der Figur dargestellt ist. Beim Transport des Blutes 5 durch die Blutkammer gelangen Blutbestandteile, wie beispielsweise Mittelmoleküle, Wasser etc. über die semipermeable Membran des Dialysators 5 auf die Seite der Dialysierfüssigkeit und werden dort abtransportiert.

Die Effektivität konvektiver Dialyseverfahren (H(D)F pre/post/mixed) (HDF = Hämodiafiltration; HF = Hämofiltration, pre = Predilution; post = Postdilution; mixed = Pre- und Postdilution) mit Highflux-Dialysatoren wird im Bereich der Mittelmoleküle wie z. B. β2m, im Wesentlichen durch die erzielbare Austauschrate $Q_{sub}$ während der Behandlung bestimmt. Der Wert $Q_{sub}$ ist der durch die Substituatpumpe 12 geförderte Substituatfluss. In der Ausführungsform gemäß Figur 5 wird das Substituat stromabwärts des Dialysators 5 dem Blut zugegeben, so dass ein HDF-Postitutions-Verfahren vorliegt.

Im Dialysator 5 wird dem Vollblutstrom $Q_b$ ein Teil des Plasma-Wasserstroms $Q_p$ entzogen, was zur Folge hat, dass das Blut eine gewisse Eindickung erfährt. In dem dargestellten Ausführungsbeispiel kann dies beispielsweise bedeuten, dass der Hämatokrit $H_{kt}$ am Ausgang des Dialysators um 10 % bis 20 % gegenüber dem am Eingang des Dialysators ansteigt.

Um das Blut wieder auf den ursprünglichen Hämatokrit zu verdünnen, wird wie in Figur 5 dargestellt stromabwärts des Dialysators 5 mittels der Pumpe 12 das entzogene Fluidvolumen kontinuierlich durch den Substituatfluss $Q_{sub}$ ersetzt. Wenn der Substituatfluss bzw. die Substituatpumpe 12 gestoppt werden muss, beispielsweise wegen eines Leitfähigkeitsalarms oder aufgrund gelegentlicher Dichtigkeitstests der Hydraulik, hat dies zur Folge, dass das in dem Dialysator 5 eingedickte, höher viskose Blut über das venöse Schlauchsystem zurück zum Patienten gelangt.

Das Bezugszeichen 13 kennzeichnet die Hydraulik bzw. den Bilanzierkreislauf für den Dialysatfluss $Q_d$.

Wie bereits ausgeführt, gelangt das Blut über eine venöse Nadel zurück zum Patienten. Stromaufwärts dieser venösen Nadel befindet sich die venöse Schlauchabsperrklemme 10 sowie stromaufwärts dazu ein optischer Detektor 8 sowie ein Luftblasendetektor 9. Des Weiteren sind zwischen diesen Detektoren und der Zufuhrleitung des Substituats ein venöser Blasenfänger mit Füllstandsdetektor 7 vorgesehen. An diesen ist ein Hydrophob-Filter 6a angeschlossen, der seinerseits mit einem venösen Drucksensor 6b zur Ermittlung des venösen Druckes $p_{ven}$ in Verbindung steht.

Gelangt das höher viskose Blut zurück zum Patienten, kann insbesondere in der venösen Nadel die höhere Viskosität des Bluts zu einem zusätzlichen Druckanstieg führen, der unter Umständen 200 mmHg und mehr betragen kann. Dies kann zu einer unnötigen Scherspannungsbelastung des Bluts führen. Der durch die höhere Viskosität des Bluts bedingte Druckanstieg ist unmittelbar am venösen Drucksensor 6b messbar.

Will man den genannten Druckanstieg und die damit verbundene Belastung des Bluts reduzieren, bestünde die Möglichkeit, während der Stillstandzeit der Substituatpumpe 12 die Blutpumpe 4 ebenfalls anzuhalten, damit kein höher viskoses Blut zum Patienten gelangt. In diesem Fall bleibt das Blut im Dialysator 5 stehen. Wenn beide Pumpen 4, 12 wieder gleichzeitig anlaufen, entsteht der genannte Hämokonzentrations-Bolus, d.h. der Bolus an verdicktem Blut nicht. Das Anhalten der Blutpumpe führt allerdings zu einer Verunsicherung des Anwenders. Darüber hinaus besteht ein Nachteil darin, dass im Falle einer länger stehenden Substituatpumpe und Blutpumpe die Gefahr besteht, dass das Blut im extrakorporalen Kreislauf koaguliert, was einen nicht akzeptablen Blutverlust zur Folge hätte.

Eine alternative Möglichkeit zur Verhinderung des Hämokonzentrations-Bolus bestünde darin, den Blutfluss $Q_b$ zu reduzieren und die Blutpumpenrate in Abhängigkeit des venösen Drucks $p_{ven}$ so zu regeln, dass der Druckanstieg ausgeglichen bzw. abgeschwächt wird. Ein solches Verfahren ist aus der WO 2009/144522 A1 bekannt.

Mittels des in Figur 5 dargestellten Drucksensors 11 zur Ermittlung des Vorfilterdrucks bzw. des Postpumpdruckes $p_{pp}$ kann ein clotting von Blut im Dialysator sowie auch die genannte hohe Hämokonzentrierung des Bluts durch eine hohe Ultrafiltration erkannt werden. Durch diesen Sensor ist es ebenfalls möglich, das Vorliegen eines Hämokonzentrations-Bolus zu erfassen. Des Weiteren kann durch diesen Sensor ein Abknicken des Blutschlauches und eine damit verbundene Druckerhöhung erfasst werden.

Ein Nachteil dieses Sensors besteht zum Einen in den durch diesen verursachten Mehrkosten und zum Anderen in oft verwendeten luftgefüllten Druckableitungen. Dadurch entsteht eine Blut-Luft-Grenzfläche, an der es zur Gerinnung kommen kann bzw. an der eine Gerinnung einsetzen kann. Darüber hinaus besteht die Gefahr, dass bei einer Undichtigkeit Blut ins Innere des Gerätes läuft, was im Hinblick auf die elektrische Sicherheit und in Bezug auf Kreuzkontaminationen mit infektiösen Substanzen über das Gerät mit entsprechenden Risiken behaftet ist.

Daher ist es anzustreben, ein Abknicken des Blutschlauches, ein clotting oder auch eine Hämokonzentrationserhöhung im Dialysator ohne einen solchen zusätzlichen, invasiven Drucksensor sicher zu erkennen.

DE 10 2010 011798 A1 betrifft ein Verfahren zum Bestimmen eines Drucks in einem Volumenstrom des Fluids unter Einsatz wenigstens einer Zentrifugalpumpe.

Der vorliegenden Erfindung liegt somit die Aufgabe zugrunde, ein Verfahren der eingangs genannten Art dahingehend weiterzubilden, dass eine Alternative zur Druckmessung durch einen Drucksensor bereitgestellt wird.

Diese Aufgabe wird erfindungsgemäß durch ein Verfahren mit den Merkmalen des Anspruchs 1 gelöst. Danach ist vorgesehen, dass zur Ermittlung des Drucks p oder des mit diesem korrelierten Parameters der Motorstrom $I_M$ des genannten Motors sowie der Blutfluss $Q_b$ oder wenigstens ein damit korrelierter Parameter gemessen wird und dass aus den gemessenen Werten der Druck p oder der mit diesem korrelierte Parameter, wie z.B. eine Druckdifferenz berechnet wird.

Der vorliegenden Erfindung liegt somit der Gedanke zugrunde, aus der Messung des Motorstroms $I_M$ oder eines damit korrelierten Parameters sowie des Blutflusses $Q_b$ oder eines damit korrelierten Parameters Rückschlüsse auf den Druck p, vorzugsweise Rückschlüsse auf den Druck p stromabwärts der Blutpumpe zu ziehen bzw. diesen Druck zu berechnen oder auch einen damit korrelierten Parameter, wie beispielsweise die Druckdifferenz über die Pumpe.

Somit ist es möglich, aus der Messung der genannten Parameterwerte den Druck zu ermitteln oder beispielsweise auch eine Druckdifferenz. Auf den Einsatz eines invasiven Drucksensors, wie er durch den Sensor 11 in Figur 5 gemäß dem Stand der Technik bekannt ist, kann verzichtet werden.

Denkbar ist es beispielsweise, dass durch das Verfahren die Druckdifferenz $\Delta p$ stromaufwärts und stromabwärts der Blutpumpe bzw. zwischen Ein- und Auslass der Pumpe, der Druck $p_{art}$ stromaufwärts der Blutpumpe und/oder der Druck $p_{pp}$ stromabwärts der Blutpumpe ermittelt wird.

In weiterer Ausgestaltung der Erfindung ist vorgesehen, dass es sich bei dem mit dem Blutfluss $Q_b$ korrelierten Parameter um die Drehzahl n der Blutpumpe und/oder um die Winkelgeschwindigkeit $\omega$ der Blutpumpe handelt. Bei dem mit dem Strom korrelierten Parameter kann es sich beispielweise um die bereitgestellte elektrische Leistung der Stromversorgung des Motors handeln.

Die Druckdifferenz stromaufwärts und stromabwärts der Blutpumpe wird gemäß der Formel $I_M = a + b \cdot Q_b + c \cdot Q_b \cdot \Delta p$ ermittelt. Diese Beziehung ergibt sich aus folgenden Überlegungen:

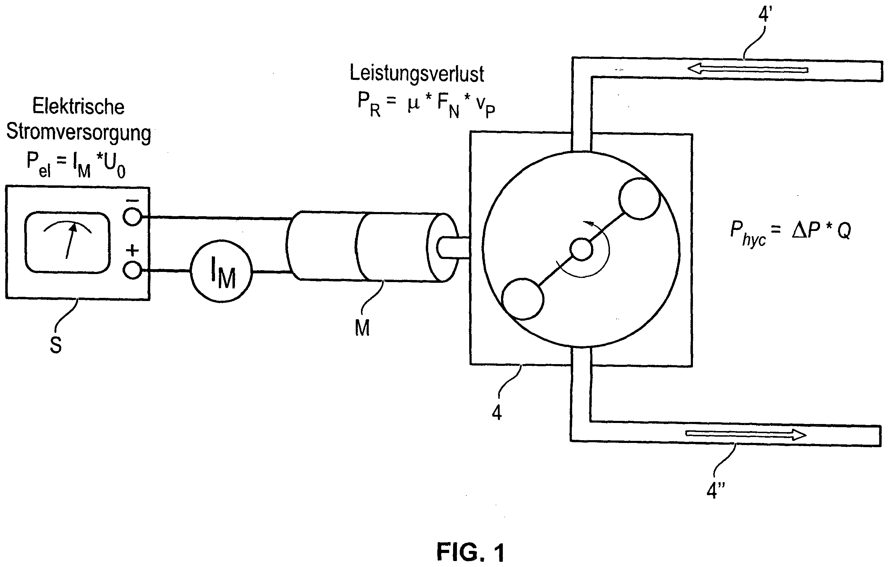

Aus Figur 1 ist mit dem Bezugszeichen der Elektromotor M einer peristaltischen Pumpe 4 sowie dessen Stromversorgung S dargestellt. Die peristaltische Pumpe ist beispielsweise an der Position des extrakorporalen Blutkreislaufs gemäß Figur 5 eingesetzt und fördert somit das Blut von einem Einlass 4' zu einem Auslass 4".

Die elektrische Leistung der Stromversorgung S $P_{el}$ teilt sich im Antrieb auf drei Anteile auf, nämlich $P_0$, $P_R$ und $P_{hyc}$. Dabei ist $P_{el}$ die von der Stromversorgung abgegebene elektrische Leistung, d. h. $I_M \cdot Uo$, wobei es sich bei $U_0$ um die Spannung handelt, die von der Spannungsversorgung abgegeben wird, wie beispielsweise 24 V.

Der Wert $P_0$ ist der konstante Leistungsverbrauch, der sich beispielsweise durch die Steuerelektronik im BLDC-Motor, Wärmeverluste, Eisenverluste etc. ergibt.

Bei dem Wert $P_R = \mu \cdot F_N \cdot v_p$ handelt es sich um Leistungsverluste, die proportional zur Geschwindigkeit $v_p$ der Pumpe sind. Dies sind beispielsweise Reibungsverluste in den Lagern der mechanisch bewegten Teile, im Getriebe und in den Rollen sowie die Kupfer-induzierten Verluste in den Leitungen.

Die Normalkraft $F_N$ in der Pumpe entsteht durch die Verpressung des Schlauches bzw. durch die Rückstellkraft des Pumpenschlauches, um die Okklusion zu erreichen. Diese kann durch verspannte Federn erzeugt werden.

Bei der Leistung $P_{hcy}$ handelt es sich um die technische Leistung, die die Pumpe an das strömende Medium, d. h. an das Blut abgibt. Diese ist bestimmt durch $P_{hyc} = Q_b$

$$\cdot \Delta p = Q_b \cdot (p_{pp} - p_{art}).$$

Alle der vorgenannten Drucke sind statische Relativdrucke.

Der Motorstrom wird vorzugsweise in Ampere, die Vorsorgungsspannung $U_0$ in Volt, beispielsweise $U_0 = 24V$, $\mu$ als effektiver Reibungskoeffizient einheitslos, $F_N$ in Newton, $v_p$ in m/s, $Q_b$ in $m^3$/s und die Druck in Pa angegeben.

Setzt man die angegebenen Beziehungen für $P_{el}$, $P_R$ und $P_{hyc}$ in die Beziehung:

$$P_{el} = P_0 + P_R + P_{hyc}$$

ein, ergibt sich folgende Beziehung:

$$I_M \cdot U_0 = P_0 + \mu \cdot F_N \cdot v_p + Q_b \cdot \Delta p.$$

Löst man diese Beziehung nach dem Motorstrom $I_M$ auf, ergibt sich:

$$I_M = P_0 / U_0 + \mu \cdot F_N \cdot v_p/U_0 + 1/U_0 \cdot Q_b \cdot \Delta p.$$

Die Geschwindigkeit $v_p$ kann durch die Drehzahl $n_p$ oder durch die Winkelgeschwindigkeit $\omega_p$ ersetzt werden:

$$v_p = 2\pi \cdot R \cdot n_p = R \cdot \omega_p.$$

Wird die Geschwindigkeit $v_p$ durch eine weitere Größe, nämlich durch den Blutfluss $Q_b$ ersetzt ($Q_b = V_s \cdot v_p/(2\pi \cdot R)$) ergibt sich schließlich

$$I_M = P_0/U_0 + \mu \cdot (F_N/U_0) \cdot (2\pi \cdot R)/V_S \cdot Q_b + 1/U_0 \cdot Q_b \cdot \Delta p.$$

Dabei bedeuten $V_S$ das Schlagvolumen der Blutpumpe pro Umdrehung [$m^3$], typischerweise 10 mL, R den Radius des Blutpumpenrotors [m], n die Drehzahl der Blutpumpe [1/s] und $\omega$ die Winkelgeschwindigkeit [rad/s].

Mithilfe der Substitutionen $a = P_0/U_0$, $b = (\mu \cdot F_N)/U_0 \cdot (2\pi \cdot R)/V_s$ und $c = 1/U_0$ ergibt sich folgende Gleichung:

$$I_M = a + b \cdot Q_b + c \cdot Q_b \cdot \Delta p \qquad \text{(Gleichung (1))}$$

Bei bekannten Parametern a, b und c kann man diese Beziehung nach der Druckdifferenz $\Delta p$ auflösen. Somit ergibt sich:

$$\Delta p = I_M/(c \cdot Q_b) - a/(c \cdot Q_b) - b/c = 1/c \cdot (I_M/Q_b - a/Q_b - b).$$

Durch diese Beziehung ist es bei Messungen des Motorstroms und des Blutflusses möglich, bei Kenntnis der Parameter a, b und c die Druckdifferenz vor und nach der Pumpe, d. h. den Wert $\Delta p$ zu errechnen.

Aus der Beziehung $\Delta p = p_{pp} - p_{art}$ kann der Druck unmittelbar nach der Blutpumpe, d. h. der Wert $p_{pp}$ berechnet werden. Der arterielle Druck $p_{art}$ wird gemessen und $\Delta p$ kann aus der vorgenannten Gleichung ermittelt werden. Somit ergibt sich:

$$p_{pp} = \Delta p + p_{art}.$$

Um die Genauigkeit der Fitfunktion zu erhöhen, kann die oben angegebene Gleichung (1), die die Relation zwischen Motorstrom, Blutfluss und Druckdifferenz wiedergibt, um höhere, auch nicht rationale Potenzen von $Q_b$ und $\Delta p$ erweitert werden:

$$I_M = \sum\sum c_{p,q} \cdot Q_b^{\ q} \cdot \Delta p^p$$

wobei die Summen über p = 0 bis m und q = 0 bis n gebildet werden, wobei p, q ∈ Q sind. Denkbar ist des Weiteren ein Verfahren zur Regelung oder Steuerung des Druckes oder eines mit dem Druck korrelierten Parameters in einem extrakorporalen Blutkreislauf eines Blutbehandlungsgerätes, insbesondere eines Dialysegerätes, wobei sich in den Blutkreislauf wenigstens eine Blutpumpe befindet, die durch wenigstens einen Motor angetrieben wird. Demgemäß ist denkbar, dass der Druck oder ein mit diesem korrelierter Parameter gemäß einem Verfahren nach einem der Ansprüche 1 bis 4 ermittelt wird und dass zum Zwecke der Regelung oder Steuerung der mittels der Pumpe geförderte Blutfluss verändert wird.

Somit ist es denkbar, die oben genannten ermittelten bzw. berechneten Druckwerte oder darauf basierende Parameter, wie beispielsweise die Druckdifferenz über die Pumpe dazu heranzuziehen, um ein Regelungsverfahren aufzusetzen, bei der die Regelgröße der Druck oder ein damit korrelierter Parameter, wie beispielsweise eine Druckdifferenz ist. Die Stellgröße wird vorzugsweise durch die Blutpumpenrate, d. h. durch den Blutfluss $Q_b$ oder einen damit korrelierten Parameter gebildet.

Denkbar ist es, dass das Verfahren die Begrenzung des Druckes oder eines mit dem Druck korrelierten Parameters dahingehend umfasst, dass ein Grenzwert nicht überschritten werden darf. Auch eine Druckregelung ist denkbar und bevorzugt. Denkbar ist es, dass ein Grenzwert für eine maximal zulässige Druckänderung vorgegeben wird, wobei diese Druckänderung nicht unmittelbar gemessen wird, sondern erfindungsgemäß über die Beobachtung des Motorstroms errechnet wird. Wird dieser Grenzwert erreicht oder überschritten, kann die eigentliche Regelung zur Konstanthaltung des Druckes oder eines damit korrelierten Parameters begonnen werden.

In bevorzugter Ausgestaltung ist vorgesehen, dass die Regelung derart durchgeführt wird, dass das Verhältnis $(I_M(t) - a)/Q_{b(t)} = (I_{M,0} - a)/Q_{b,0} = const$ konstant bleibt. Bei den Werten $I_{M,0}$ und $Q_{b,0}$ handelt es sich um Startwerte des Motorstroms und des Blutflusses zu einem Zeitpunkt vor Eintritt einer Störung, insbesondere vor Überschreiten eines Grenzwertes. Die vorliegende Erfindung betrifft des Weiteren eine Blutbehandlungsvorrichtung mit wenigstens einem extrakorporalen Blutkreislauf, in dem wenigstens eine Blutpumpe angeordnet ist, die durch wenigstens einen Elektromotor angetrieben wird. Es ist wenigstens ein Mittel zur Messung des Motorstroms $I_M$ und wenigstens ein Mittel zur Messung des Blutflusses $Q_b$ oder jeweils damit korrelierter Parameter vorgesehen. Die Blutbehandlungsvorrichtung umfasst wenigstens eine Rechen-, Steuer- oder Regelungseinheit, die so ausgebildet ist, dass sie basierend auf den durch die Mittel bereitgestellten Messwerten das Verfahren gemäß einem der Ansprüche 1 bis 4 durchführt.

Vorzugsweise handelt es sich bei der Blutpumpe um eine peristaltische Pumpe. Deren Motor wird vorzugsweise durch einen Elektromotor gebildet und besonders bevorzugt durch einen Gleichstrommotor und gegebenenfalls durch einen bürstenlosen Gleichstrommotor.

In dem extrakorporalen Kreislauf befindet sich vorzugsweise wenigstens ein Dialysator, der wenigstens eine in Betrieb des Kreislaufs von Blut durchströmte Kammer aufweist. Zudem kann wenigstens eine im Betrieb von Dialysierflüssigkeit durchströmte Kammer vorgesehen sein.

In einer weiteren Ausgestaltung der Erfindung ist wenigstens eine Substituatpumpe vorgesehen, die über eine Leitung stromaufwärts und/oder stromabwärts des Dialysators dem Blut in dem extrakorporalen Kreislauf eine Substitutionsflüssigkeit zuführt.

Schließlich kann vorgesehen sein, dass stromabwärts der Blutpumpe außer eines venösen Drucksensors kein Drucksensor vorgesehen ist und dass vorzugsweise stromabwärts der Blutpumpe und stromaufwärts eines in dem extrakorporalen Blutkreislauf angeordneten Dialysators kein Drucksensor angeordnet ist.

Die Blutbehandlungsvorrichtung ist vorzugsweise mit Mitteln ausgeführt, die geeignet sind, einen, mehrere oder alle der jeweiligen Verfahrensschritte gemäß der Verfahrensansprüche durchzuführen.

Weitere Einzelheiten und Vorteile der Erfindung werden anhand eines in den Zeichnungen dargestellten Ausgangsbeispiels näher erläutert. Es zeigen:

Figur 1: eine schematische Ansicht der peristaltischen Blutpumpe mit Motor und Leistungsversorgung,

Figur 2: die Abhängigkeit des Motorstroms von Blutfluss und Druckdifferenz über die Pumpe,

Figur 3: ein Beispiel für eine Stromversorgung mit Shuntwiderstand,

Figur 4: ein Beispiel für eine High-Side-Strommessung mit 12 Bit-ADC und

Figur 5: eine schematische Darstellung eines extrakorporalen Kreislaufes eines Dialysegerätes gemäß dem Stand der Technik.

Wie bereits oben ausgeführt, kann erfindungsgemäß ein Druck bzw. ein mit diesem korrelierter Parameter, wie beispielsweise die Druckdifferenz vor und nach der Blutpumpe auf der Grundlage der Messung des Motorstroms und des Blutflusses oder damit korrelierter Größen berechnet werden.

Dieser berechnete Wert kann herangezogen werden, um eine Druckregelung vorzunehmen. Auf diese Weise kann verhindert werden, dass es zu Druckspitzen bzw. zu Druckwerten kommt, die gegebenenfalls zu einer Schädigung des Blutes führen können.

In erfindungsgemäßer Ausgestaltung wird die Beziehung zwischen dem Motorstrom, dem Blutfluss und der Druckerhöhung über die Pumpe durch die folgende Beziehung wiedergegeben:

$$I_M = a + b \cdot Q_b + c \cdot Q_b \cdot \Delta p.$$

Löst man diese Gleichung nach der Druckdifferenz $\Delta p$ auf, ist ersichtlich, dass basierend auf den Messgrößen $I_M$ und $Q_b$ die Druckdifferenz ermittelt werden kann. Auf diese Weise lässt sich auch bei Messung des arteriellen Druckes der Druck stromabwärts der Blutpumpe berechnen.

Hinsichtlich der Anordnung der entsprechenden Drucksensoren wird insoweit auf die Figur 5 verwiesen. Die Figur 5 bzw. eine derartige Anordnung eines extrakorporalen Blutkreislaufes ist auch für die vorliegende Erfindung geeignet und von dieser mit umfasst, wobei vorzugsweise vorgesehen ist, dass erfindungsgemäß auf den Drucksensor 11, d.h. auf den Drucksensor stromabwärts der Blutpumpe 4 verzichtet wird.

Die Darstellung gemäß Figur 1 verdeutlicht, dass aus den dort dargestellten Größen $P_{el}$, $P_R$ und $P_{hyc}$ bzw. aus dem Zusammenhang $P_{el} = P_0 + P_R + P_{hyc}$ die vorgenannte Beziehung aus Motorstrom, Blutfluss und Druckdifferenz über die Pumpe ermittelt werden kann.

In Figur 1 ist der Motorstrom ebenfalls mit dem Bezugszeichen $I_M$ angegeben.

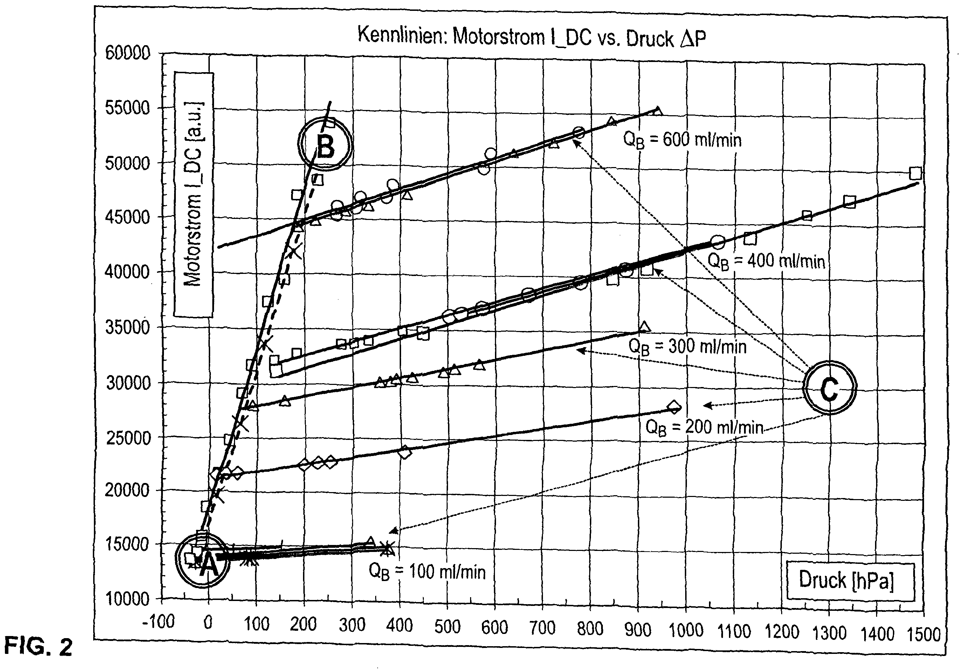

Figur 2 zeigt die Abhängigkeit des Motorstroms als Funktion des Blutflusses und des Druckes bzw. der Druckdifferenz über die Pumpe basierend auf der oben angegebenen Beziehung $I_M = f(Q_b; \Delta p)$.

Wie dies aus der Figur 2 hervorgeht, steigt entsprechend dieser Beziehung der Motorstrom bei konstantem Blutfluss mit der Druckdifferenz an.

Der Punkt A in Figur 2 ist durch eine Druckerhöhung $\Delta p = 0$ gekennzeichnet. Hier liegt auch kein Blutfluss vor, so dass sich für den Motorstrom der Wert a ergibt.

Die Strecke A-B ist gekennzeichnet durch eine Druckerhöhung über die Pumpe von ungefähr 0, wobei der Blutfluss von 100 bis 700 ml/min variiert. In diesem Fall ist die Änderung des Motorstroms $\Delta I_M$ entlang der Strecke A-B $\Delta I_M = b \cdot Q_b$.

Die dargestellten Strecken C sind jeweils durch konstante Werte des Blutflusses (100; 200; 300; 400; 500 und 600 ml/min) gekennzeichnet. Hier ergibt sich die Beziehung $\Delta I_M = c \cdot Q_b \cdot \Delta p$.

Will man nicht nur eine Druckmessung vornehmen, sondern eine Konstantregelung des Druckes, kann gewählt werden, welcher Druck, beispielsweise $\Delta p$, $p_{pp}$ bestimmt werden soll und auf einen konstanten Wert bzw. einen Sollwertbereich geregelt werden soll. Die Regelung erfolgt vorzugsweise durch Einstellung der Blutpumpenrate bzw. Blutgeschwindigkeit $Q_b$.

Die Ausgangsbasis bildet die oben genannte Beziehung:

$$I_M = a + b \cdot Q_b + c \cdot Q_b \cdot \Delta p.$$

Das totale Differenzial dieser Gleichung ergibt $dI_M = b \cdot dQ_b + c \cdot dQ_b \cdot \Delta_p + c \cdot Q_b \cdot d\Delta p$. Die partielle Ableitung bei konstantem Druck $\Delta p$ ergibt:

$$(\partial I_M / \partial Q_b)_{\Delta p} = b + c \cdot \Delta p = const.$$

Nach der partiellen Ableitung bei konstantem Druck $\Delta p$ ergibt sich bei einem Vergleich mit Gleichung (1) für $\Delta p = const$:

$$(I_M - a) / Q_b = b + c \cdot \Delta p = const.$$

Die Empfindlichkeit $E_{\Delta p}$ des Motorstroms gegenüber der Druckänderung von $\Delta p$ erhält man aus der partiellen Ableitung bei konstantem Blutfluss $Q_b$:

$$E_{\Delta p} = \Delta I_M / \Delta(\Delta p) = (\partial I_M / \partial \Delta p)_{Qb} = c \cdot Qb.$$

Ein möglicher Ablauf zum Betrieb des Systems bzw. zur Regelung des Druckes besteht darin, dass im ungestörten Zustand, also bei normalem Betrieb der Pumpen und insbesondere auch der Substituatpumpe, die Messgrößen $I_M$ und $Q_b$ in regelmäßigen Abständen als Startwerte $I_{M,0}$ und $Q_{b,0}$ gemerkt bzw. gespeichert werden.

Tritt während der Behandlung eine Störung auf, kann zunächst geprüft werden, ob eine maximal zulässige Druckänderung erreicht wird. Diese maximale Druckänderung kann durch $\Delta(\Delta p)_{GW}$ wiedergegeben werden. Dazu wird die Änderung des Motorstroms beobachtet. Denkbar ist es, dass wenn sich der Motorstrom um mehr als

$$\Delta I_M = c \cdot Q_b \cdot \Delta(\Delta p)_{GW}$$

ändert, die aktive Regelungsphase begonnen wird, in der auf einen Druckwert oder auf einen Druckwertbereich geregelt wird.

Unter Berücksichtigung der aufgezeichneten bzw. gemerkten Startwerte für den Motorstrom und den Blutfluss kann der Blutfluss nun durch die Regelung bzw. durch eine Regelungseinheit so verändert werden, dass das Verhältnis $(I_M(t)-a)/Q_b(t)=(I_{M,0}-a)/Q_{b,0}$=const konstant bleibt.

Denkbar ist es weiterhin, dass nach einem Timeout oder nach einem bestimmten geförderten Blutvolumen der Ablauf beendet wird. Der Blutfluss wird dann wieder auf den ursprünglichen Wert $Q_{b,0}$ gesetzt.

Wie dies aus der oben genannten Beziehung zur Konstanthaltung des Druckes hervorgeht, wird der Parameter b nicht benötigt und der Parameter c nur zu einem Triggerzeitpunkt benötigt, nämlich dann wenn festgestellt werden soll, ob die zulässige Druckänderung überschritten wurde oder nicht. Dieser Wert c muss nicht sehr genau sein. Die Genauigkeit des Parameters a spielt nur bei relativ großen Änderungen eine Rolle.

Von den eingangs genannten Parametern a, b und c muss nur ein Systemparameter, nämlich a bestimmt werden. Es reicht aus, den Motorstrom $I_M$ bei niedrigem Fluss z.B. $Q_b$<100 ml/Minute und bei niedrigem Druck $\Delta p$ zum Beispiel im Rahmen der Vorbereitung oder beim Füllen des Schlauchsystems zu messen.

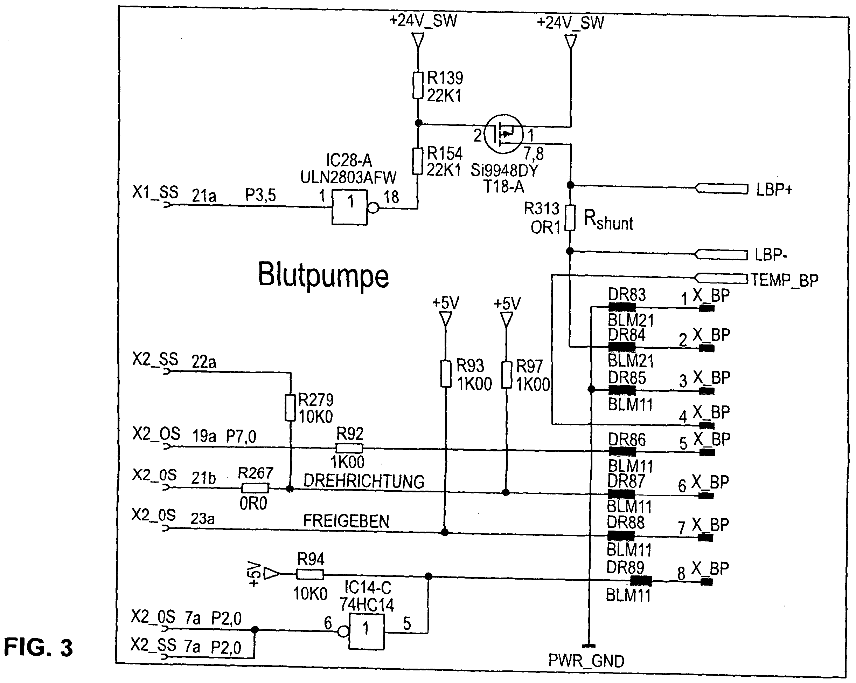

Figur 3 zeigt ein Beispiel für eine Stromversorgung mit Shuntwiderstand. In diesem Fall wird in die 24 Volt-Versorgungsleitung des Motors ein Shuntwiderstand $R_{Shunt}$=0,1 $\Omega$ eingefügt. Der Spannungsabfall an dem Shunt wird mit einem High-Side-Stromfüller in einen Ausgangsstrom bzw. -spannung mit Masse-(GND)Bezug umgewandelt und über einen ADC digitalisiert. Die weitere Signalverarbeitung erfolgt in einem Mikrocontroller.

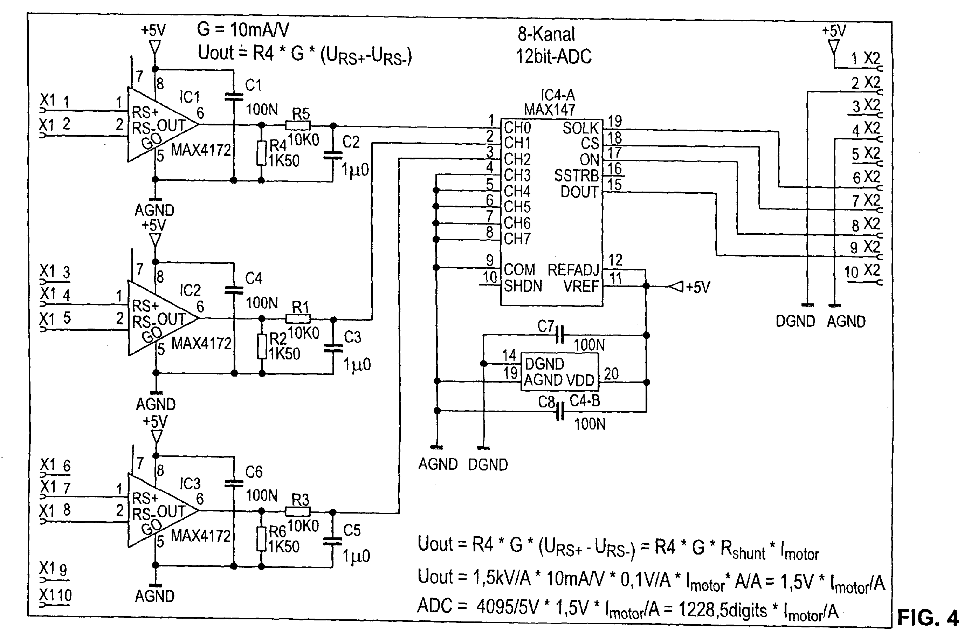

Dies ergibt sich aus der Darstellung gemäß Figur 4.

Die Fitparameter a, b und c lassen sich wie folgt bestimmen: Die Fitparameter werden so gewählt, dass die Summe der Fehlerquadrate S minimal wird. Die Summe der Fehlerquadrate ist $S = \sum (I_{M,i} - a - b \cdot Q_{b,i} - c \cdot Q_{b,i} \cdot \Delta p_i)^2$, wobei die Summe in dieser Beziehung und in den folgenden Gleichungen jeweils über i gebildet wird.

Dazu wird der Extremwert von S bestimmt:

$$\partial S/\partial a = 0 \rightarrow \sum (I_{M,i} - a - b \cdot Q_{b,i} - c \cdot Q_{b,i} \cdot \Delta p_i) = 0$$

$$\rightarrow \sum I_{M,i} = N \cdot a + b \cdot \sum Q_{b,i} + c \cdot \sum (Q_{b,i} \cdot \Delta p_i)$$

$$\rightarrow y_1 = a \cdot s_{1,1} + b \cdot s_{2,1} + c \cdot s_{3,1}$$

$$\partial S/\partial b = 0 \rightarrow \sum (I_{M,i} - a - b \cdot Q_{b,i} - c \cdot Q_{b,i} \cdot \Delta p_i) \cdot Q_{b,i} = 0$$

$$\rightarrow \sum I_{M,i} \cdot Q_{b,i} = a \cdot \sum Q_{b,i} + b \cdot \sum Q_{b,i}^2 + c \cdot \sum (Q_{b,i}^2 \cdot \Delta p_i)$$

$$\rightarrow y_2 = a \cdot s_{1,2} + b \cdot s_{2,2} + c \cdot s_{3,2}$$

$$\partial S/\partial c = 0 \rightarrow \sum (I_{M,i} - a - b{\cdot}Q_{b,i} - c{\cdot}Q_{b,i}{\cdot}\Delta p_i) \cdot Q_{b,i}{\cdot}\Delta p_i = 0$$

$$\rightarrow \sum (I_{M,i}{\cdot}Q_{b,i}{\cdot}\Delta p_i) = a{\cdot}\sum (Q_{b,i}{\cdot}\Delta p_i) + b{\cdot}\sum(Q_{b,i}{}^2{\cdot}\Delta p_i) + c{\cdot}\sum (Q_{b,i}{\cdot}\Delta p_i)^2$$

$$\rightarrow y_3 = a{\cdot}s_{1,3} + b{\cdot}s_{2,3} + c{\cdot}s_{3,3}$$

Die drei Gleichungen zur Bestimmung von $y_1$, $y_2$ und $y_3$ können zu einer Vektor-Gleichung zusammengefasst werden: $y = a{\cdot}s_1 + b{\cdot}s_2 + c{\cdot}s_3$

Mit Hilfe der Determinanten det ( ) erhält man die Lösungen:

$a = \det (y; s_2; s_3) / \det (s_1; s_2; s_3)$

$b = \det (s_1; y; s_3) / \det (s_1; s_2; s_3)$

$c = \det (s_1; s_2; y) / \det (s_1; s_2; s_3)$

**Patentansprüche**

1. Verfahren zur Ermittlung des Druckes p oder eines mit dem Druck p korrelierten Parameters in einem extrakorporalen Blutkreislauf einer Blutbehandlungsvorrichtung, insbesondere eines Dialysegerätes, wobei sich in dem Blutkreislauf wenigstens eine Blutpumpe (4) befindet, die durch wenigstens einen Motor (M) angetrieben wird, wobei zur Ermittlung des Druckes p oder des mit diesem korrelierten Parameters der Motorstrom $I_M$ des genannten Motors (M) sowie der Blutfluss $Q_b$ oder ein damit korrelierter Parameter gemessen wird und aus den gemessenen Werten der Druck p oder der mit diesem korrelierte Parameter berechnet wird, **dadurch gekennzeichnet, dass** die Druckdifferenz $\Delta p$ stromaufwärts und stromabwärts der Pumpe (4) gemäß der Formel $I_M = a + b * Q_b + c * Q_b * \Delta p$ ermittelt wird.

2. Verfahren nach Anspruch 1, **dadurch gekennzeichnet, dass** durch das Verfahren die Druckdifferenz $\Delta p$ stromaufwärts und stromabwärts der Blutpumpe (4), der Druck $p_{art}$ stromaufwärts der Blutpumpe (4), der Druck $p_{pp}$ stromabwärts der Blutpumpe (4) ermittelt wird.

3. Verfahren nach Anspruch 1 oder 2, **dadurch gekennzeichnet, dass** es sich bei dem mit dem Blutfluss $Q_b$ korrelierten Parameter um die Drehzahl n der Blutpumpe (4) und/oder um die Winkelgeschwindigkeit $\omega$ der Blutpumpe (4) handelt.

4. Verfahren nach einem der vorhergehenden Ansprüche, **dadurch gekennzeichnet, dass** der Druck $p_{pp}$ stromabwärts der Blutpumpe (4) gemäß der Formel $p_{pp} = \Delta_P + p_{art}$ ermittelt wird.

5. Blutbehandlungsvorrichtung mit wenigstens einem extrakorpoalen Blutkreislauf, in dem wenigstens eine Blutpumpe (4) angeordnet ist, die durch wenigstens einen Motor (M) angetrieben wird, wobei wenigstens ein Mittel zur Messung des Motorstroms $I_M$ und wenigstens ein Mittel zur Messung des Blutflusses $Q_b$ oder damit korrelierter Parameter vorgesehen sind, **dadurch gekennzeichnet, dass** die Blutbehandlungsvorrichtung wenigstens eine Rechen-, Steuer- oder Regelungseinheit aufweist, die derart ausgebildet ist, dass sie basierend auf den durch die Mittel bereitgestellten Meßwerten das Verfahren gemäß einem der Ansprüche 1 bis 4 durchführt.

6. Blutbehandlungsvorrichtung nach Anspruch 5, **dadurch gekennzeichnet, dass** es sich bei der Blutpumpe (4) um eine peristaltische Pumpe (4) handelt und/oder dass es sich bei dem Motor (M) um einen mit Gleichstrom betriebenen und vorzugsweise um einen bürstenlosen Gleichstrommotor handelt.

7. Blutbehandlungsvorrichtung nach Anspruch 5 oder 6, **dadurch gekennzeichnet, dass** in dem extrakorporalen Kreislauf wenigstens ein Dialysator (5) vorgesehen ist, der wenigstens eine im Betrieb von Blut durchströmte Kammer aufweist.

8. Blutbehandlungsvorrichtung nach Anspruch 7, **dadurch gekennzeichnet, dass** der Dialysator (5) wenigstens eine im Betrieb von Diaysierflüssigkeit durchströmte Kammer aufweist.

9. Blutbehandlungsvorrichtung nach einem der Ansprüche 5 bis 8 **dadurch gekennzeichnet, dass** wenigstens eine Substituat-Pumpe (12) vorgesehen ist, die über eine Leitung stromaufwärts und/oder stromabwärts des Dialysators (5) dem Blut in dem extrakorporalen Kreislauf eine Substitutionsflüssigkeit zuführt.

10. Blutbehandlungsvorrichtung nach einem der Ansprüche 5 bis 9, **dadurch gekennzeichnet, dass** stromabwärts der Blutpumpe (4) außer eines venösen Drucksensor (6b) kein Drucksensor vorgesehen ist und dass vorzugsweise stromabwärts der Blutpumpe (4) und stromaufwärts eines in dem extrakorporalen Blutkreislauf angeordneten Dialysators (5) kein Drucksensor angeordnet ist.

**Claims**

1. A method of determining the pressure p or a parameter correlated with the pressure p in an extracorporeal blood circuit of a blood treatment apparatus, in particular of a dialyzer, wherein at least one blood pump (4) which is driven by at least one motor (M) is located in the blood circuit, wherein the motor current $I_M$ of the said motor (M) and the blood flow $Q_b$ or a parameter correlated therewith is measured for determining the pressure p or the parameter correlated therewith and the pressure p or the parameter correlated therewith is calculated from the measured values, **characterized in that** the pressure difference $\Delta p$ is determined upstream and downstream of the pump (4) in accordance with the formula $I_M = a + b * Q_b + c * Q_b * \Delta p$.

2. The method in accordance with claim 1, **characterized in that** using the method the pressure difference $\Delta p$ is determined upstream and downstream of the blood pump (4), the pressure $p_{art}$ upstream of the blood pump (4) and the pressure $p_{pp}$ downstream of the blood pump (4).

3. The method in accordance with claim 1 or claim 2, **characterized in that** the parameter correlated with the blood flow $Q_b$ is the speed n of the blood pump (4) and/or the angular speed $\omega$ of the blood pump (4).

4. The method in accordance with one of the preceding claims, **characterized in that** the pressure $p_{pp}$ is determined downstream of the blood pump (4) in accordance with the formula $p_{pp} = \Delta p + p_{art}$.

5. A blood treatment apparatus having at least one extracorporeal blood circuit in which at least one blood pump (4) is arranged which is driven by at least one motor (M), wherein at least one means is provided for measuring the motor current $I_M$ and at least one means is provided for measuring the blood flow $Q_b$ or parameters correlated therewith, **characterized in that** the blood treatment apparatus has at least one calculating unit, control unit or regulation unit which is configured such that it carries out the method in accordance with one of claims 1 to 4 on the basis of the measured values provided by the means.

6. The blood treatment apparatus in accordance with claim 5, **characterized in that** the blood pump (4) is a peristaltic pump (4) and/or **in that** the motor (M) is a DC motor operated with DC current, preferably a brushless DC motor.

7. The blood treatment apparatus in accordance with claim 5 or claim 6, **characterized in that** at least one dialyzer (5) which has at least one chamber flowed through by blood in operation is provided in the extracorporeal circuit.

8. The blood treatment apparatus in accordance with claim 7, **characterized in that** the dialyzer (5) has at least one chamber flowed through by dialysis fluid in operation.

9. The blood treatment apparatus in accordance with one of the claims 5 to 8, **characterized in that** at least one substituate pump (12) is provided which supplies a substitution fluid to the blood in the extracorporeal circuit via a line upstream and/or downstream of the dialyzer (5).

10. The blood treatment apparatus in accordance with one of the claims 5 to 9, **characterized in that** no pressure sensor is provided, with the exception of a venous pressure sensor (6b), downstream of the blood pump (4) and **in that** no pressure sensor is preferably arranged downstream of the blood pump (4) and upstream of a dialyzer (5) arranged in the extracorporeal blood circuit.

**Revendications**

1. Procédé destiné à déterminer la pression p ou un paramètre corrélé avec la pression p dans un circuit sanguin extracorporel d'un dispositif de traitement du sang, en particulier un appareil de dialyse dans lequel se trouve au moins une pompe à sang (4) située dans le flux sanguin et entraînée par au moins un moteur (M), le courant du moteur IM dudit moteur (M) ainsi que le flux sanguin $Q_b$ ou un paramètre ainsi corrélé étant mesurés pour déterminer la pression p ou le paramètre corrélé avec celle-ci et la pression p ou le paramètre corrélé avec celle-ci sont calculés à partir des valeurs mesurées de la pression p ou du paramètre corrélé avec celle-ci, **caractérisé en ce que** le différentiel de pression $\Delta p$ en amont et en aval de la pompe (4) est déterminé selon la formule $I_M = a + b * Q_b + c * Q_b * \Delta p$.

2. Procédé selon la revendication 1, **caractérisé en ce que** le procédé permet de déterminer le différentiel de pression $\Delta p$ en amont et en aval de la pompe à sang (4), la pression $p_{art}$ en amont de la pompe à sang (4), la pression $p_{pp}$ en aval de la pompe à sang (4).

3. Procédé selon la revendication 1 ou 2, **caractérisé en ce qu'**il s'agit dans le cas du paramètre corrélé avec le débit sanguin $Q_b$ de la vitesse de rotation n de la pompe à sang (4) et/ou de la vitesse angulaire $\omega$ de la pompe à sang (4).

4. Procédé selon l'une quelconque des revendications précédentes, **caractérisé en ce que** la pression $p_{pp}$ en aval de la pompe à sang (4) est déterminée selon la formule $p_{pp} = \Delta p + p_{art}$.

5. Dispositif de traitement du sang avec au moins un flux sanguin extracorporel, dans lequel au moins une pompe à sang (4) est agencée, laquelle est entraînée par au moins un moteur (M), **caractérisé en ce qu'**au moins un moyen destiné à mesurer le courant du moteur $I_M$ et au moins un moyen destiné à mesurer le débit sanguin $Q_b$ ou les paramètres corrélés sont prévus et **en ce que** le dispositif de traitement du sang présente au moins une unité de calcul, de commande ou de régulation, configurée de sorte à réaliser le procédé selon l'une quelconque des revendications 1 à 4 en se fondant sur les valeurs mesurées fournies par les moyens.

6. Dispositif de traitement du sang selon la revendication 5, **caractérisé en ce qu'**il s'agit pour la pompe à sang (4) d'une pompe péristaltique (4) et/ ou en qu'il s'agit pour le moteur (M) d'un moteur actionné par courant continu ou de préférence d'un moteur à courant continu sans balais.

7. Dispositif de traitement du sang selon la revendication 5 ou 6, caractérisé ce en qu'au moins un dialyseur (5) est prévu dans le circuit sanguin extracorporel, lequel présente au moins une chambre traversée par le sang durant le fonctionnement.

8. Dispositif de traitement du sang selon la revendication 7, **caractérisé en ce que** le dialyseur (5) présente au moins une chambre traversée par le fluide de dialyse durant le fonctionnement.

9. Dispositif de traitement du sang selon l'une quelconque des revendications 5 à 8, **caractérisé en ce qu'**au moins une pompe à liquide de restitution (12) est prévue, laquelle alimente le sang dans le circuit extracorporel en liquide de substitution via une conduite située en amont et/ou en aval du dialyseur (5).

10. Dispositif de traitement du sang selon l'une quelconque des revendications 5 à 9, **caractérisé en ce qu'**aucun capteur de pression n'est prévu en aval de la pompe à sang (4) excepté le capteur de pression veineux (6b) et **en ce qu'**aucun capteur de pression n'est agencé de préférence en aval de la pompe à sang (4) et en amont d'un dialyseur (5) agencé dans le circuit sanguin extracorporel.

**FIG. 1**

Elektrische
Stromversorgung
$$P_{el} = I_M * U_0$$

Leistungsverlust
$$P_R = \mu * F_N * v_P$$

$$P_{hyc} = \Delta P * Q$$

FIG. 2

FIG. 3

FIG. 4

$U_{out} = R4 * G * (U_{RS+} - U_{RS-})$

$G = 10mA/V$

$U_{out} = R4 * G * (U_{RS+} - U_{RS-}) = R4 * G * R_{shunt} * I_{motor}$

$U_{out} = 1,5kV/A * 10mA/V * 0,1V/A * I_{motor} * A/A = 1,5V * I_{motor}/A$

$ADC = 4095/5V * 1,5V * I_{motor}/A = 1228,5 digits * I_{motor}/A$

EP 2 986 332 B1

FIG. 5
Stand der Technik

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009144522 A1

- DE 102010011798 A1